# EUROPEAN PATENT APPLICATION

(11) **EP 3 121 599 A1**
(43) Date of publication of application: **25.01.2017**
(21) Application number: 15178138.2
(22) Date of filing: 23.07.2015
(51) Int. Cl.: G01N 33/68

(54) **ANALYSIS FOR ATHEROSCLEROSIS**

(71) Applicant: Medizinische Hochschule Hannover, 30625 Hannover (DE)
(72) Inventor: Baerlecken, Dr., Niklas, 30171 Hannover (DE); Witte, Prof., Torsten, 30559 Hannover (DE); Ernst, Dr., Diana, 30625 Hannover (DE)
(74) Representative: Taruttis, Stefan Georg

(57) **Abstract**

The invention provides a process for analysis of the presence of atherosclerosis in a patient sample, and preferably identifies a form of atherosclerosis that correlates to an increased risk of the patient to develop rheumatoid arthritis (RA), connective tissue diseases, vasculitis, and/or spondyloarthritis, and/or myocardial infarction and/or apoplexy.

## Description

The present invention relates to a process for analysis of the presence of atherosclerosis in a patient sample. Further, the invention relates to a process for analysis of the risk for myocardial infarction and/or apoplexy.

### State of the art

Atherosclerosis is a pathologic process causing disease of the coronary, cerebral, and peripheral arteries. Atherosclerosis may start already in childhood, and at an age between 30 and 34 years, 20 % of men and 8 % of women have advanced coronary atheromata. The pathogenesis of atherosclerosis is not entirely clear yet, but endothelial dysfunction, inflammation, and the traditional risk factors of hypertension, diabetes, dyslipidemia, and smoking play important roles. Atherosclerosis is also increased in patients suffering from chronic inflammatory disorders such as rheumatoid arthritis, spondyloarthritis, vasculitis and connective tissue diseases. Patients with these diseases have a reduced life expectancy due to increased mortality by cardiovascular complications.

Atherosclerosis is generally asymptomatic until the atherosclerotic plaques reduce the luminal diameter of involved arteries, and angina pectoris or other ischemic complications such as apoplexy or intermittent claudication may occur. In addition, ruptures of vulnerable plaque may cause immediate problems, such as apoplexy, acute coronary syndromes or myocardial infarction.

Extensive efforts have been made to identify these rupture-prone "vulnerable plaques" in order to be able to treat patients prone to sudden death early. CT angiography appears to be appropriate, but is costly and is associated with radiation exposure. Therefore, screening tools to identify patients at high risk for problems caused by vulnerable plaques are not available yet.

Within the vulnerable plaque, macrophages accumulate and contain macrophages large amounts of free cholesterol. The macrophages turn into foamy cells, and eventually die from apoptosis. The macrophages are activated by cytokines, and produce matrix metalloproteinases (MMPs).

MMPs are important in the pathogenesis of atherosclerosis by participating in vascular remodeling, smooth muscle cell migration, and plaque disruption. MMP-14 is detected at high levels in the atherosclerotic plaque and is induced by the transcription factor MAZI (SAF-1), which is and coexpressed with MMP-14 in the macrophages present in the atherosclerotic plaques.

Bataller et al., Ann Neurol 2003, 53: 123-127 describes the MAZ protein as an autoantigen of Hodgkin's disease and of paraneoplastic dysfunction.

Ray et al., The Journal of Immunology 2004, 172: 5006-5015 describes that the inflammation-responsive transcription factor SAF-1 when overexpressed in cultivated cells causes a growth-arresting signal which then stops cellular proliferation via activating expression of p21.

Ray et al., Circ Res. 2004, 95 : 1082-1090 describes that it is known that matrix metalloproteinases (MMPs) play an important role in the pathogenesis of atherosclerosis and in plaque disruption, and show that increased binding of SAF-1 to the MMP-14 gene induces transcriptional upregulation of this gene.

### Object of the invention

It is an object of the invention to provide for an analytical process that identifies the presence of atherosclerosis from a patient sample, especially from blood serum, but also from blood, plasma, saliva, urine or other biomaterials, and preferably identifies a form of atherosclerosis that correlates to an increased risk of cardiovascular complications. The invention is also suitable to detect atherosclerosis leading to complications such as myocardial infarction and/or apoplexy in patients with chronic inflammatory disorders such as rheumatoid arthritis, connective tissue diseases, vasculitis, and/or spondyloarthritis.

### Description of the invention

The invention achieves the object by the features of the claims and especially by providing a process for analysis of the presence of atherosclerosis in a patient sample, and preferably identifies a form of atherosclerosis that correlates to an increased risk of the patient to develop rheumatoid arthritis (RA), connective tissue diseases, vasculitis, and/or spondyloarthritis, and/or myocardial infarction and/or apoplexy. The process is based on the detection of auto-antibody specific for MAZI (Myc-associated zinc finger protein, alternatively named serum amyloid A-activating factor-1(SAF-1), purine-binding transcription factor (PUR-1)) (anti-MAZI auto-antibody) in the sample. MAZI preferably has the amino acid sequence given in UniProtKB as P56270 (MAZ_HUMAN). The process optionally includes or consists of the analysis for the presence of the auto-antibody specific for MAZI for detecting the presence of atherosclerosis, myocardial infarction and/or apoplexy and/or rheumatoid arthritis, especially in patients with chronic inflammatory disorders, e.g. in patients with rheumatoid arthritis.

It has been found that the presence of anti-MAZI auto-antibody in a sample correlates with presence of atherosclerosis in the patient, and especially correlates with atherosclerosis having vulnerable plaques. The presence of vulnerable plaques is believed to correlate with an increased risk for myocardial infarction and/or apoplexy.

The correlation of anti-MAZI auto-antibody with atherosclerosis, including in patients with rheumatoid arthritis, connective tissue diseases, vasculitis, and/or spondyloarthritis, and with an increased risk for myocardial infarction and/or apoplexy is surprising, because MAZI (also termed SAF-1) is known as a transcription factor, hence a protein localized in the cell nucleus.

The process for analysis comprises or consists of the steps of
- determining the concentration of anti-MAZI auto-antibody in a patient sample, which preferably is blood serum, but can also be another body fluid such as blood, plasma, urine or saliva,
- comparing the determined concentration of anti-MAZI auto-antibody to the concentration determined in samples of healthy persons, preferably of the same age,
- wherein an elevated concentration of anti-MAZI auto-antibody in the patient sample indicates presence of atherosclerosis, including in patients with rheumatoid arthritis, , connective tissue diseases, vasculitis, and/or of spondyloarthritis, and preferably indicates an increased risk for myocardial infarction and/or apoplexy.

An elevated concentration of the anti-MAZI auto-antibody is e.g. a concentration higher than the mean concentration in samples of healthy persons, higher than the mean concentration in samples from an arbitrary group of persons, e.g. non-preselected persons, e.g. blood donors, or preferably higher than the mean concentration in samples from persons who are diagnosed as being free from atherosclerosis, including atherosclerosis in patients with rheumatoid arthritis, connective tissue diseases, vasculitis, spondyloarthritis, myocardial infarction and/or apoplexy. The mean concentration of the anti-MAZI auto-antibody in serum is used as a cut-off value, with concentrations elevated above the cut-off value indicating atherosclerosis with significance, especially atherosclerosis associated with vulnerable plaques. Optionally, the cut-off value is the mean concentration of the anti-MAZI auto-antibody plus 1-fold or 2-fold the value of the standard deviation.

A concentration of anti-MAZI auto-antibody in the patient sample higher than the cut-off value indicates presence of atherosclerosis, especially of atherosclerosis associated with vulnerable plaques, and preferably indicates an increased risk for myocardial infarction and/or apoplexy.

Optionally, the cut-off value is age-dependent, e.g. increasing with increasing age of the patient. For this embodiment, the cut-off value used is optionally determined in healthy persons of an age group of a period +/- 5 years or +/- 2 to 3 years around the age of the patient from whom the sample originates, or in age groups of 45-55 years, 56-65 years, and 66 years and above.

Preferably, the process in addition to detecting the concentration of anti-MAZI auto-antibody in a patient sample by determination of the amount of antibody binding to MAZI protein comprises a parallel determination of the binding of a reference sample containing a pre-determined concentration of anti-MAZI auto-antibody to MAZI protein. More preferably, the reference sample contains a pre-determined concentration of anti-MAZI auto-antibody that is equivalent to the cut-off value.

In addition or in the alternative, the process can optionally comprise the step of adding a pre-determined amount of anti-MAZI auto-antibody to an aliquot of the patient sample and/or adding a pre-determined amount of soluble MAZI protein to an aliquot of the patient sample, and determining the amount of anti-MAZI antibody in these samples, preferably in an ELISA. In an embodiment comprising the step of adding a pre-determined amount of anti-MAZI antibody to an aliquot of the patient sample, the determined concentration of anti-MAZI antibody is reduced by the amount of added anti-MAZI antibody. In an embodiment comprising the addition of soluble MAZI protein to an aliquot of the patient sample, the determined concentration of anti-MAZI antibody is increased by an amount that is equimolar to the amount of added soluble MAZI protein. Preferably, the analysis of a patient sample aliquot to which a pre-determined amount of anti-MAZI antibody and/or of a patient sample aliquot to which a pre-determined amount of MAZI protein has been added, is made in parallel to the analysis of the serum sample.

An anti-MAZI antibody can be produced by standard processes for producing antibody, e.g. using vaccination of an experimental animal with purified MAZI protein and isolating serum from the animal, and/or by the hybridoma technique for generating an antibody-producing cell line from fusion products of spleen cells of a MAZI-vaccinated animal with a proliferating cell line, or by expression of an anti-MAZI antibody in a cultivated cell or micro-organism using generally known genetic engineering processes.

The analysis of a sample for determination of the concentration of anti-MAZI auto-antibody can be by an ELISA, using MAZI protein or peptides as a capture agent, e.g. recombinant or native or alternatively peptides derived from the MAZI sequence, that is immobilized on a support as a capture agent for the anti-MAZI auto-antibody, followed by removal of unbound sample components and detection of bound antibody, e.g. using a labelled anti-human antibody as a detection antibody, followed by removal of unbound detection antibody and measurement of the label, e.g. direct measurement of the label or measurement of a reaction product, e.g. a colour, produced by the label from an added colour precursor.

As an alternative to an ELISA using microtiter plates, other procedures using reaction tubes, beads, biochips etc. may be performed. The immobilization of the capture agent can be obtained by adsorption, covalent binding or by high affinity interactions (such as streptavidin/biotin or hapten/anti-hapten-antibodies). The immobilized capture agent may be used in a form bound to the solid phase already at the start of the reaction, or may be immobilized later on in the test procedure.

The procedure may be performed in fluid phase, for example in a reaction tube, or in a dry system, for example on a test strip. Such methods are in principle known and can be adapted for detection of the anti-MAZI autoantibody.

The capture agent may carry a detectable group as a label, also termed direct labeling, or may be able to bind to a detectable group, also termed indirect labeling. The label may be from any suitable detection system, for examples enzymes, metal or latex particles, with luminescent or fluorescent groups. Preferentially, enzymes such as peroxidase, beta galactosidase or alkaline phosphatase are selected as label, and an ELISA will be performed.

The anti-MAZI auto-antibody can comprise all antibody classes and subclasses, and accordingly, the process can comprise determination of anti-MAZI auto-antibody of any antibody class or subclass.

The invention is now described in greater detail by way of examples and with reference to the figures showing in
- Fig. 1 ELISA results for anti-MAZI auto-antibody in sera of patients diagnosed by whole body CT scans to have atherosclerosis (+) and of patients without diagnosed atherosclerosis (-),
- Fig. 2 ELISA results for anti-MAZI auto-antibody in sera of patients who were examined for coronary artery disease by coronary artery catherization,
- Fig. 3 ELISA results anti-MAZI auto-antibody in sera of patients who were diagnosed for atherosclerosis by whole body CT, separately for age groups, and in
- Fig. 4 ELISA results for anti-MAZI auto-antibody in sera of rheumatoid arthritis patients who were diagnosed for ischemic heart disease and by cancer patients, in whom the presence (PET_AORTA+) or absence (PET_AORTA-) of atherosclerosis of the aorta had been examined by PET CT.

An ELISA was carried out using blood serum as the sample. 0.1 µg recombinant MAZI protein in 100µl PBS (phosphate buffered saline, pH), purchased from Abnova, Taiwan, was adsorbed for immobilization onto each well of a 96-well microtiter plate (Maxisorb, Nunc, Denmark) by incubation overnight at 5°C. The supernatant was then removed and 0.1 % casein in PBS was used for blocking for 30 min at room temperature. Sera were diluted 1:100 in 0.1% casein in PBS-T (Tris-phosphate with 20% Tween20) for 30 min at room temperature, then washed three times using 200µl TBS-T. Detection was by addition of 100µl of a goat anti-human IgG coupled to HRP (horse radish peroxidase) as the labelled detection antibody, purchased from Jackson ImmunoResearch Europe Ltd. at a dilution of 2:10 000 or at 2µl in 10ml 0.1% casein in PBS, and after incubating for 30 min, wells were washed three times using PBS-T, then the colour reaction was made using the colour precursor TMB (3,3',5,5'-tetramethylbenzidine) for at maximum 15 min according to the manufacturer's (Thermo Fisher Scientific, Denmark) instructions. Colour measurement was at 450nm using an ELISA reader.

In non-preselected blood donor samples obtained from a blood bank, a positive reaction for anti-MAZI auto-antibody was found in 4 of 39 individuals.

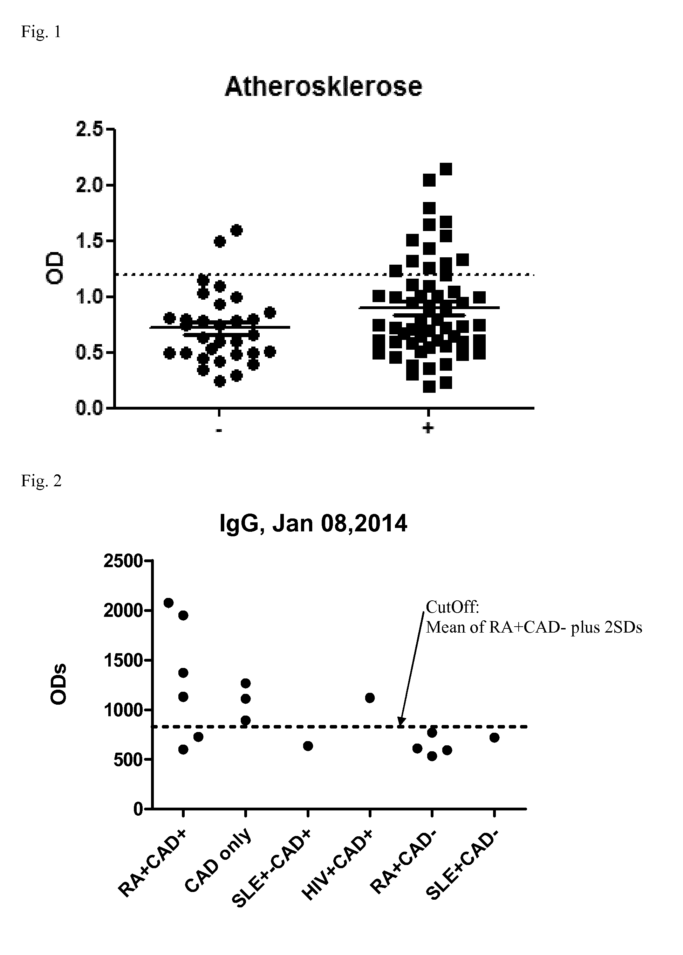

Analysis of serum from 87 patients that were examined for atherosclerosis using PET-CT (positron-emission tomography-computerized tomography) showed that patients who were diagnosed in PET-CT as having atherosclerosis had concentrations of the anti-MAZI auto-antibody above the cut-off concentration to a significantly higher extent than patients who were diagnosed in PET-CT as not having atherosclerosis. Fig. 1 shows the concentrations of anti-MAZI auto-antibody as OD measured in the ELISA for patients who were diagnosed in PET-CT as having atherosclerosis (+), and for patients who were diagnosed in PET-CT as not having atherosclerosis (-). The cut-off value (broken line) of OD 1.2 was determined in parallel in 39 arbitrary sera of blood donors.

Analysis of the data of Fig. 1 showed that the mean concentration of the anti-MAZI auto-antibody of all patients diagnosed as having atherosclerosis (+) is significantly higher than the mean concentration of all patients who were diagnosed in PET-CT as not having atherosclerosis (-). The concentration of the anti-MAZI auto-antibody of patients diagnosed as having atherosclerosis (+) is significantly higher also when analysing the values higher than the cut-off value by Fisher's exact test using a fourfold table.

Fig. 2 shows ELISA results in measured OD for the anti-MAZI auto-antibody of patients who were examined using a heart catheter due to suspected myocardial infarction. Patients who were diagnosed positive for coronary artery disease (CAD+ and CAD only) were found to have anti-MAZI auto-antibody to a significantly higher proportion than patients who were diagnosed negative for coronary artery disease (CAD-). Fig. 2 indicates concomitant diseases, namely rheumatoid arthritis (RA), Sjögren's syndrome (SLE), and HIV infection (HIV). The cut-off value was chosen as the mean concentration of the anti-MAZI auto-antibody in sera from the patients who were diagnosed as negative for coronary artery disease (CAD-) but having RA plus two-fold the standard-deviation (2SDs).

These results show that the concentration of anti-MAZI auto-antibody is essentially only above the cut-off value in patients diagnosed positive for coronary artery disease, showing that an elevated concentration of the anti-MAZI auto-antibody is characteristic for coronary artery disease as one of the manifestations of atherosclerosis.

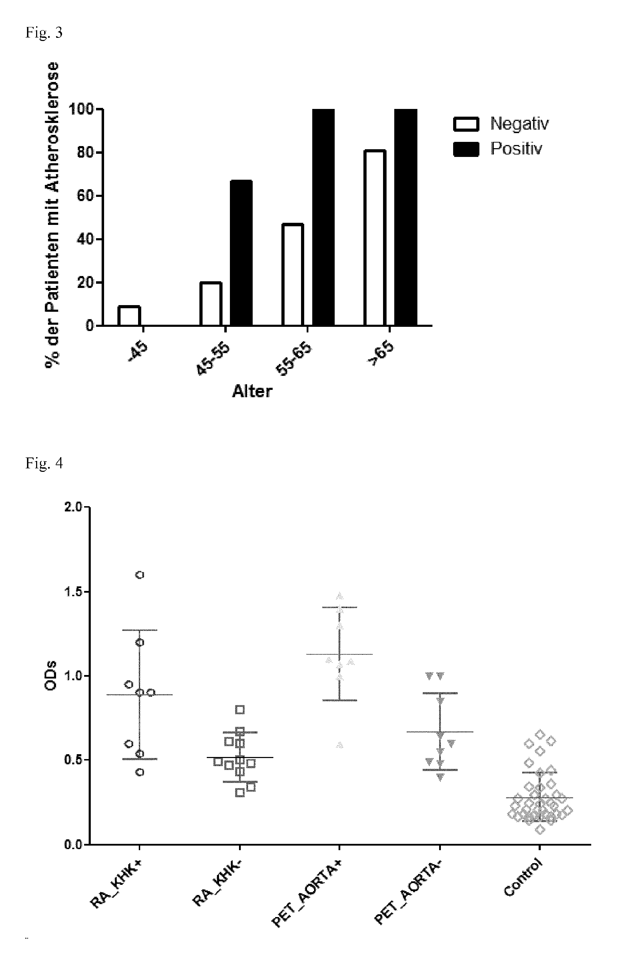

Fig. 3 shows the proportion of patients from Figure 1 who were diagnosed for atherosclerosis in different age groups, for those patients in whom a concentration of anti-MAZI auto-antibody was found below the cut-off value of 1.2 OD (Negativ, empty columns) and for those patients in whom a concentration above the cut-off value (Positiv, black columns) was found in the ELISA. These data show that the presence of the anti-MAZI auto-antibody is a strong indicator for presence of atherosclerosis, especially at age groups 45 to 55 years and 55 to 65 years.

Fig. 4 shows ELISA results in OD for anti-MAZI auto-antibody in patients previously diagnosed positive for RA (RA Patients) and diagnosed positive for presence of ischemic heart disease (KHK+) or diagnosed negative for ischemic heart disease (KHK-). This shows that an ischemic heart disease correlates with a significant higher concentration of the anti-MAZI auto-antibody at least in RA patients. In addition, patients with atherosclerosis of the aorta (diagnosed by CT) (PET_AORTA+) also had significantly higher serum concentrations of anti-MAZI auto-antibody compared to patients without atherosclerosis of the aorta (diagnosed by CT) (PET_AORTA-).

## Claims

1. Process for analysis of the presence of atherosclerosis, **characterized by** determining the concentration of anti-MAZI auto-antibody in a patient sample.

2. Process according to claim 1, **characterized in that** an elevated concentration of anti-MAZI auto-antibody is indicative of atherosclerosis and/or of an increased risk for myocardial infarction and/or of an increased risk for apoplexy.

3. Process according to one of the preceding claims, **characterized in that** the sample originates form a patients with a chronic inflammatory disorder, e.g. a disorder selected from rheumatoid arthritis, connective tissue diseases, vasculitis, and/or spondyloarthritis, and/or myocardial infarction and/or apoplexy.

4. Process according to one of the preceding claims, **characterized by** comparing the concentration of the anti-MAZI auto-antibody determined in the patient sample to the mean concentration of anti-MAZI auto-antibody determined in samples of an arbitrary group of persons, or in samples from persons who are diagnosed as being free from atherosclerosis, rheumatoid arthritis connective tissue diseases, vasculitis, spondyloarthritis, myocardial infarction and/or apoplexy.

5. Process according to one of the preceding claims, **characterized in that** atherosclerosis and/or an increased risk for myocardial infarction and/or apoplexy is determined for a concentration of the anti-MAZI auto-antibody in the patient sample elevated over the mean concentration of anti-MAZI auto-antibody determined in samples of an arbitrary group of persons, or in samples from persons who are diagnosed as being free from atherosclerosis, rheumatoid arthritis, rheumatoid spondylitis, connective tissue diseases, vasculitis, spondyloarthritis, myocardial infarction and/or apoplexy.

6. Process according to one of the preceding claims, **characterized by** analysing in parallel an aliquot of the patient sample to which a pre-determined amount of anti-MAZI antibody has been added and/or by analysing in parallel an aliquot of the patient sample to which a pre-determined amount of MAZI protein has been added.

7. Process according to one of the preceding claims, **characterized by** the patient sample being a human body fluid selected from blood serum, blood, urine and saliva.

8. Process according to one of the preceding claims, **characterized by** MAZI comprising or consisting of the amino acid sequence given in UniProtKB as P56270 (MAZ_HUMAN).

9. Process according to one of the preceding claims, **characterized in that** MAZI protein is used as a capture agent for the anti-MAZI auto-antibody.

10. Test kit for use in a process according to one of the preceding claims, comprising MAZI protein and a labelled anti-human antibody.

11. Use of MAZI protein having the amino acid sequence given in UniProtKB as P56270 (MAZ_HUMAN) as a capture agent for anti-MAZI auto-antibody in the analysis of a sample for atherosclerosis and/or for an increased risk for myocardial infarction and/or apoplexy and/or rheumatoid arthritis.
